# EUROPEAN PATENT SPECIFICATION

(11) **EP 3 944 824 B1**
(45) Date of publication and mention of the grant of the patent: **17.12.2025**
(21) Application number: 21187342.7
(22) Date of filing: 23.07.2021
(51) Int. Cl.: A61B 17/11, A61B 17/115, A61B 17/00, A61B 90/00

(54) **SYSTEMS FOR CONTROLLING A SURGICAL STAPLING INSTRUMENT**
SYSTEME ZUR STEUERUNG EINES CHIRURGISCHEN KLAMMERINSTRUMENTS
SYSTÈMES DE COMMANDE D'UN INSTRUMENT D'AGRAFAGE CHIRURGICAL

(30) Priority: 27.07.2020 US 202063056746 P; 02.07.2021 US 202117366122
(43) Date of publication of application: 02.02.2022
(73) Proprietor: Covidien LP, Mansfield, MA 02048 (US)
(72) Inventor: STRASSNER, Haley E., Hamden CT Connecticut 06517 (US); KOLLAR, Charles R, Washington DC District of Columbia 20007 (US); VALENTINE, David E., Hamden CT Connecticut 06518 (US); HART, Alexander J., Tolland CT Connecticut 06084 (US); DELBO, James P., Danville PA Pennsylvania 17821 (US)
(74) Representative: Maschio & Soames IP Ltd

(56) References cited:
- EP-A1- 2 591 744
- EP-A2- 3 505 089
- US-A1- 2019 200 989
- US-A1- 2019 200 998

## Description

### CROSS-REFERENCE TO RELATED APPLICATIONS

The present application claims the benefit of and priority to U.S. Provisional Patent Application Serial No. 63/056,746, filed on July 27 2020.

### FIELD

This disclosure relates generally to powered surgical stapling instruments, and more particularly, to a method for controlling surgical stapling instruments based upon staple formation range and to a surgical stapling instrument for performing the method.

### BACKGROUND

Anastomosis is the surgical joining of separate hollow organ sections. Typically, an anastomosis procedure follows surgery in which a diseased or defective section of an organ is removed, and the remaining end sections of the organ are joined via a surgical stapling instrument. Depending on the desired anastomosis procedure, the remaining end sections may be joined by circular or side-to-side organ reconstruction methods, for instance.

In a circular anastomosis procedure, the remaining end sections of the organ are joined by means of a surgical stapling instrument which drives a circular array of staples through the remaining end sections and simultaneously cores any tissue interior of the driven circular array of staples to free a tubular passage within the organ. Staples delivered during the circular anastomosis procedure should be formed such that the contents of the gastrointestinal tracts do not leak into the abdominal or thoracic cavity.

A continuing need exists for a stapling instrument that can distinguish between staple formations that may prevent leaks and staple formations that are not sufficiently closed. EP 3505089A2 relates to a surgical stapling instrument that includes an end effector configured to clamp a tissue; a cutting member; a motor coupled to the cutting member, the motor configured to move the cutting member between a first position and a second position; and a control circuit coupled to the motor, the control circuit configured to: sense a parameter associated with clamping of the end effector; and control the motor to adjust a torque applied to the cutting member by the motor.

US 2019/200998A1 relates to a method of adjusting a staple parameter of a surgical stapling instrument. The method comprising: determining, by a control circuit of the surgical stapling instrument, a first stroke length for a first staple driver of the surgical stapling instrument to drive a first row of staples of a circular stapling head assembly of the surgical stapling instrument; detecting, by the control circuit, a malformed staple in the first row of staples; adjusting, by the control circuit, the staple parameter, based on the detection of the malformed staple; and determining, by the control circuit, a second stroke length for a second staple driver of the surgical stapling instrument to drive a second row of staples of the circular stapling head assembly.

US 2019/200989A1 relates to improved surgical systems configurable to staple tissue, surgical devices configured to staple tissue, and related methods. EP 2591744A1 relates to forceps including an end effector assembly having first and second jaw members.

### SUMMARY

The invention is defined in independent claims 1 and 8. Certain optional features of the invention are defined in the dependent claims. In accordance with the disclosure, a computer-implemented method for controlling a surgical stapling instrument for stapling tissue includes advancing a pusher towards an anvil assembly of the surgical stapling instrument from a first position to a second position, the pusher configured to eject staples from a staple cartridge of the surgical stapling instrument, determining whether the pusher stopped advancing towards the anvil assembly prior to the second position, measuring a force of staple compression of a staple being ejected from the staple cartridge by the pusher, and determining if the force of staple compression is outside of a predetermined range.

In an aspect, the method further includes entering a tissue cutting mode of the surgical stapling instrument in response to the force of staple compression based on a predetermined acceptable range of staple compression.

In another aspect, the force of staple compression may be measured by a strain gauge.

In yet another aspect, the force of staple compression may be measured based on a current of a motor configured to advance the pusher.

In an aspect, the method may further include preventing staple firing in response to the force of staple compression being greater than the predetermined range.

In another aspect, the method may further include displaying a warning in response to the force of staple compression being greater than the predetermined range.

In yet another aspect, the displayed warning may include a warning to inspect a surgical site and/or to unclamp tissue.

In still yet another aspect, the method may further include retracting the pusher.

In still yet another aspect, the method may further include generating an audio warning in response to the force of staple compression being greater than the predetermined range.

In still yet another aspect, the method may further include determining whether a functionally closed staple formation has been achieved.

In accordance with aspects of the disclosure, a surgical stapling instrument includes an anvil assembly including an anvil head and an anvil center rod extending proximally from the anvil head, a reload assembly including a pusher and an annular staple cartridge including a plurality of staples. The pusher is configured to eject staples from the annular staple cartridge. The surgical stapling instrument further includes a processor and a memory. The memory includes instructions stored thereon, which, when executed, cause the surgical stapling instrument to advance the pusher towards the anvil assembly from a first position to a second position, determine if the pusher stopped advancing towards the anvil assembly prior to the second position, measure a force of staple compression of a staple being ejected from the annular staple cartridge by the pusher, in response to the pusher having stopped advancing towards the anvil assembly, and determine if the force of staple compression is outside of a predetermined range.

In an aspect, the instructions, when executed by the processor, may further cause the surgical stapling instrument to enter a tissue cutting mode of the surgical stapling instrument in response to the force of staple compression based on a predetermined acceptable range of staple compression.

In another aspect, the force of staple compression is measured by a strain gauge.

In yet another aspect, the force of staple compression is measured based on a current of a motor configured to advance the pusher.

In still yet another aspect, the instructions, when executed by the processor, may further cause the surgical stapling instrument to prevent staple firing in response to the force of staple compression being greater than the predetermined range.

In still yet another aspect, the instructions, when executed by the processor, may further cause the surgical stapling instrument to display a warning on a display if the force of staple compression is greater than the predetermined range

In still yet another aspect, the displayed warning may include a warning to inspect a surgical site and/or to unclamp tissue.

In still yet another aspect, the instructions, when executed by the processor, may further cause the surgical stapling instrument to retract the pusher.

In still yet another aspect, the instructions, when executed by the processor, may further cause the surgical stapling instrument to generate an audio warning in response to the force of staple compression being greater than the predetermined range.

In accordance with other aspects of the disclosure, a non-transitory computer-readable medium stores instructions which, when executed by a processor, cause the processor to perform a method for controlling a surgical stapling instrument, including: advancing a pusher towards an anvil assembly of the surgical stapling instrument from a first position to a second position, the pusher configured to eject staples from a staple cartridge of the surgical stapling instrument; determining whether the pusher stopped advancing towards the anvil assembly prior to the second position; measuring a force of staple compression of a staple being ejected from the staple cartridge by the pusher; and determining if the force of staple compression is outside of a predetermined range.

### BRIEF DESCRIPTION OF DRAWINGS

Systems and methods for controlling surgical stapling instruments for clamping and stapling are disclosed herein with reference to the drawings, wherein:
FIG. 1 is a perspective view of a surgical stapling instrument in accordance with the disclosure;
FIG. 2A is a side cross-sectional view taken through a proximal portion of an adapter assembly of the surgical stapling instrument shown in FIG. 1;
FIG. 2B is a cross-sectional view taken through the distal portion of the adapter assembly and the tool assembly of the surgical stapling instrument shown in FIG. 1;
FIG. 2C is a cross-sectional view taken through the distal portion of the handle assembly of the surgical stapling instrument shown in FIG. 1;
FIG. 3A is a block diagram of a controller provided in accordance with the disclosure and configured for use with the surgical system of FIG. 1;
FIG. 3B is a block diagram of the handle assembly, the adapter assembly, and the reload assembly of the surgical system of FIG. 1 in accordance with the disclosure; and
FIG. 4 is a flowchart of a method for controlling a surgical stapling instrument for stapling in accordance with the disclosure; and
FIGS. 5A and 5B are illustrations of a staple in accordance with the disclosure and configured for use with the surgical system of FIG. 1.

### DETAILED DESCRIPTION

The disclosed surgical device will now be described in detail with reference to the drawings in which like reference numerals designate identical or corresponding elements in each of the several views. However, it is to be understood that the aspects of the disclosure are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as front, rear, upper, lower, top, bottom, distal, proximal, and similar terms are used to assist in understanding the description and are not intended to limit the disclosure.

This disclosure is directed to a surgical stapling instrument that controls stapling of tissue based in part on determination of a closure state of the staple, in particular, distinguishing between staple formations that are sufficient and prevent leaks and staple formations that are insufficient.

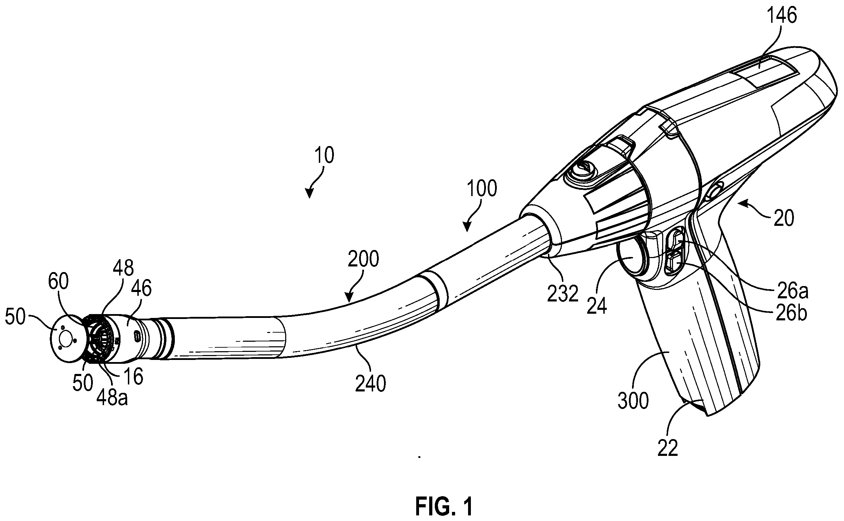

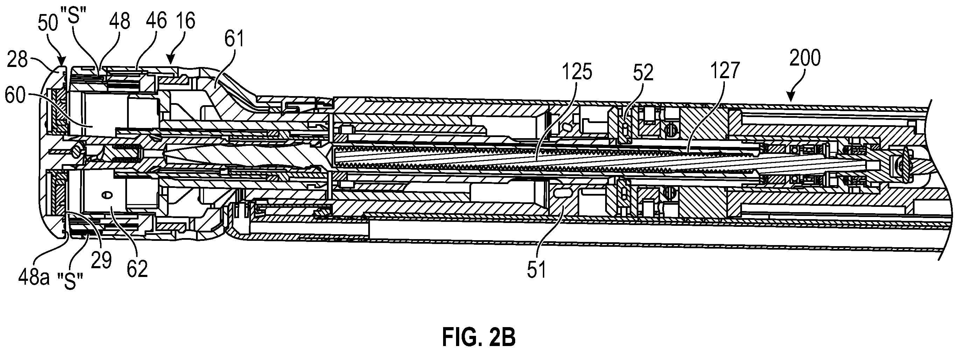

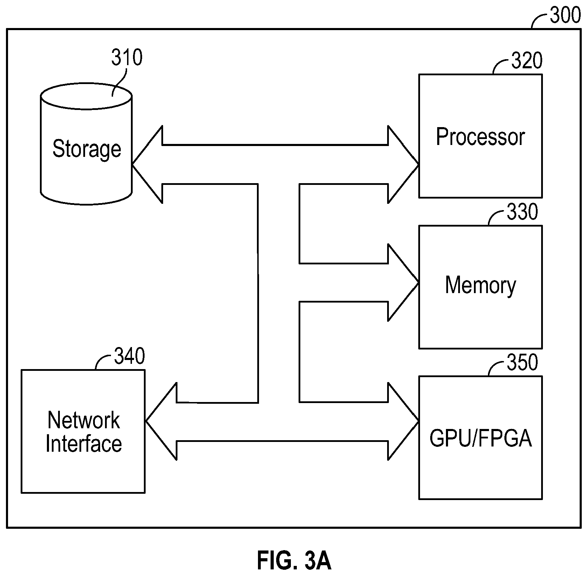

FIG. 1 illustrates a surgical stapling instrument shown generally as stapling instrument 10. The stapling instrument 10 is a circular stapling instrument and includes a handle assembly 20, an adapter assembly 100 that extends distally from the handle assembly 20, a reload assembly 16 that is supported on a distal portion of the adapter assembly 100, an anvil assembly 50 that is operatively coupled to the adapter assembly 100, and a controller 300 (FIG. 3A) that is supported within the handle assembly 20. The reload assembly 16 supports an annular staple cartridge 48 that includes a plurality of staples (not shown). The anvil assembly 50 includes an anvil head 28 that includes a staple forming surface 29 (FIG. 2B) that defines staple forming pockets 48a (FIG. 2B) and is movable in relation to the staple cartridge 48 between open and clamped positions.

The handle assembly 20 is illustrated as a powered assembly and includes a grip 22, an actuation button 24 for controlling firing of staples (not shown) from the annular staple cartridge 48 of the reload assembly 16, and approximation buttons 26a, 26b for controlling axial displacement of the anvil assembly 50 towards and away from the reload assembly 16 between the open and clamped positions. For a detailed description of the structure and function of exemplary powered handle assemblies, reference may be made to U.S. Patent Application Publication Nos. 2020/0015820 and 2019/0343517. Although the disclosure illustrates a powered assembly, it is envisioned that advantages of the disclosure, as described in detail below, are also applicable to robotically actuated surgical instruments.

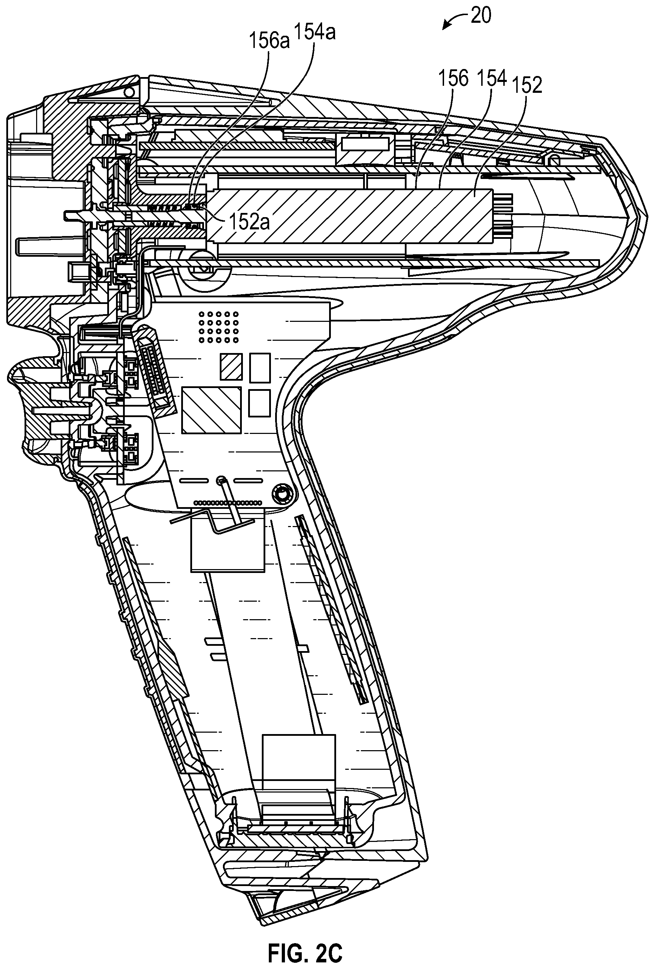

The handle assembly 20 may include an electrical assembly including a strain gauge 51 (FIG. 2B) that is configured to measure strain imparted on a lead screw 125, which is also indicative of the mechanical load on a motor (e.g., motors 152, 154, 156 of FIG. 2C) of the stapling instrument 10 due to staple formation when staples "S" are fired from the staple cartridge 48 of reload assembly 16 against anvil assembly 50.

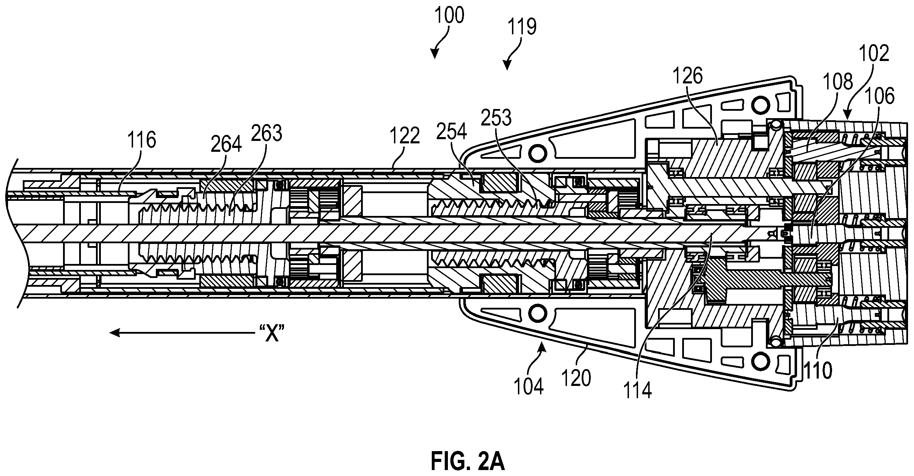

FIG. 2A illustrates the adapter assembly 100 that includes a first drive shaft 106, a second drive shaft 108, and a third drive shaft 110 that are coupled to drive shafts 152a, 154a, 156a of the motors 152, 154, 156 (FIG. 2C) supported within the handle assembly 20 (FIG. 1) when the adapter assembly 100 is coupled to the handle assembly 20 to control the various functions of the stapling instrument 10 (e.g., clamping, stapling, and/or cutting tissue). The drive shaft 106 in the adapter assembly 100 is coupled to a drive assembly 114 by gears to control movement of the anvil assembly 50 in relation to the staple cartridge 48 between open and clamped positions. The drive shaft 108 of the adapter assembly 100 is coupled to a drive assembly 119 by gears to control movement of a pusher assembly 61 (FIG. 2B) within the reload assembly 16 (FIG. 1) to control firing of staples from the staple cartridge 48 (FIG. 1). The drive shaft 110 of the adapter assembly 100 is coupled to a drive assembly 116 by gears to control cutting of tissue. Each of the drive assemblies 114, 116, 119 (FIG. 2A) includes a screw and a nut, not described in detail herein, in which the nut is driven in relation to the screw to effect longitudinal movement of the screw.

FIG. 1 illustrates the reload assembly 16, which is supported on a distal portion of the outer tube 122 of the adapter assembly 100 and includes a shell housing 46 that supports the staple cartridge 48. In aspects of the disclosure, the staple cartridge 48 defines annular rows of staple receiving pockets 48a which receive staples "S". In some aspects of the disclosure, the reload assembly 16 is releasably coupled to the distal portion of the tubular shaft (not shown) to facilitate the replacement of the annular staple cartridge 48 after each use to facilitate reuse of the surgical instrument 10. For a detailed description of exemplary aspects of a powered handle assembly and a releasable adapter assembly, reference may be made to U.S. Patent No. 10,085,744.

Each of the staple receiving pockets 48a (FIG. 2B) of the staple cartridge 48 supports a staple (not shown) that can be fired from the staple cartridge 48 via actuation of the actuation button 24 of the handle assembly 20. The shell housing 46 of the reload assembly 16 defines an annular cavity 60. The annular cavity 60 supports the staple pusher 61 (FIG. 2B) and an annular knife 62 that are coupled to the drive assembly 119 (FIG. 2A) and the knife driver nut 264 of the drive assembly 116 (FIG. 2A), respectively, such that the staple pusher 61 and the annular knife 62 are movable in relation to the staple cartridge 48 to eject the staples "S" from the staple cartridge 48 and to dissect or cut tissue positioned within an annulus defined by the staple cartridge 48. When the staples "S" are fired from the staple cartridge 48, the staples "S" are driven into and formed within the staple forming pockets 29a (FIG. 2B) of the staple forming surface 29 of the anvil head 28 of the anvil assembly 50.

As described above, the first, second, and third shafts are 106, 108, and 110 of the adapter assembly 100 are coupled to the powered handle assembly 20 by motor shafts 152a, 154a, 156a that are coupled to motors 152, 154, 156 (FIG. 2C) within the handle assembly 20 by gear assemblies (not shown). Rotation of the motor shafts 152a, 154a, 156a by the motors 152, 154, 156 are controlled by the controller 300 such that the motors 152, 154, 156 move drives the shafts 106, 108, and 110 to move the drive assembly 114, the drive assembly 119, and the drive assembly 116 through predetermined strokes to move the anvil assembly 50 in relation to the staple cartridge 48 from the open to the clamped position to define a predetermined tissue gap between the anvil assembly 50 and the staple cartridge 48, to advance the pusher 61 within the shell housing 46 to eject staples "S" from the staple cartridge 48, and to advance the knife carrier (not shown) within the shell housing 46 to cut tissue. The predetermined strokes are calculated from reference positions, which are based on the rotational position of the motor drive shafts 152a, 154a, 156a within the handle assembly 20.

Drive assembly 119 (FIG. 2A) includes a staple lead screw 253 and a staple driver nut 254 in which the staple driver nut 254 is driven in relation to the staple lead screw 253 to effect longitudinal movement of the staple lead screw 253. Drive assembly 116 (FIG. 2A) includes a knife lead screw 263 and a knife driver nut 264 in which the knife driver nut 264 is driven in relation to the knife lead screw 263 to effect longitudinal movement of the knife lead screw 263.

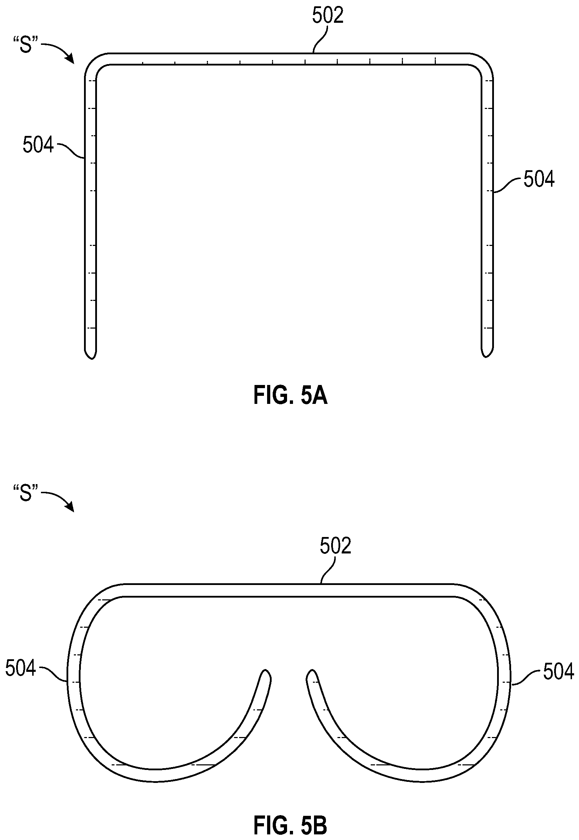

When the surgical stapling instrument 10 is fired, the staple legs 504 (FIGS. 5A and 5B) are received within the concavities (not shown) defined in the respective staple forming pockets 48a (FIG. 2B) of the anvil assembly 50. As the staple legs 504 move into the concavities, the legs 504engage the staple forming surfaces (not shown) of the staple forming pockets 48a (FIG. 2B) and are formed.

The handle assembly 20 may include a sensor such as the strain gauge 51 (FIG. 2B) that communicates with the controller 300 (FIG. 3A) and is configured to determine the load on a motor of the surgical stapling instrument 10 resulting from tissue being clamped between the anvil assembly 50 and the staple cartridge 48. This determination is used to determine a force of compression on the tissue that is clamped between the anvil assembly 50 and the staple cartridge 48.

FIG. 3A illustrates the controller 300, in accordance with the disclosure, which includes a processor 320 that is connected to a computer-readable storage medium or a memory 330. The computer-readable storage medium or memory 330 may be a volatile type memory, *e.g.,* RAM, or a non-volatile type memory, *e.g.,* flash media, disk media, *etc.* In various aspects of the disclosure, the processor 320 may be another type of processor such as, without limitation, a digital signal processor, a microprocessor, an ASIC, a graphics processing unit (GPU), a field-programmable gate array (FPGA), or a central processing unit (CPU). In certain aspects of the disclosure, network inference may also be accomplished in systems that have weights implemented as memristors, chemically, or other inference calculations, as opposed to processors.

In aspects of the disclosure, the memory 330 can be random access memory, read-only memory, magnetic disk memory, solid-state memory, optical disc memory, and/or another type of memory. In some aspects of the disclosure, the memory 330 can be separate from the controller 300 and can communicate with the processor 320 through communication buses of a circuit board and/or through communication cables such as serial ATA cables or other types of cables. The memory 330 includes computer-readable instructions that are executable by the processor 320 to operate the controller 300. The memory 330 may include volatile (e.g., RAM) and non-volatile storage configured to store data, including software instructions for operating the handle assembly 20. In other aspects of the disclosure, the controller 300 may include a network interface 340 to communicate with other computers or to a server. A storage device 310 may be used for storing data.

In aspects of the disclosure, the strain gauge 51 (FIG. 2B) is coupled to the processor, and the disclosed method is run on the controller 300 or on a user device, including, for example, on a mobile device, an IoT device, or a server system.

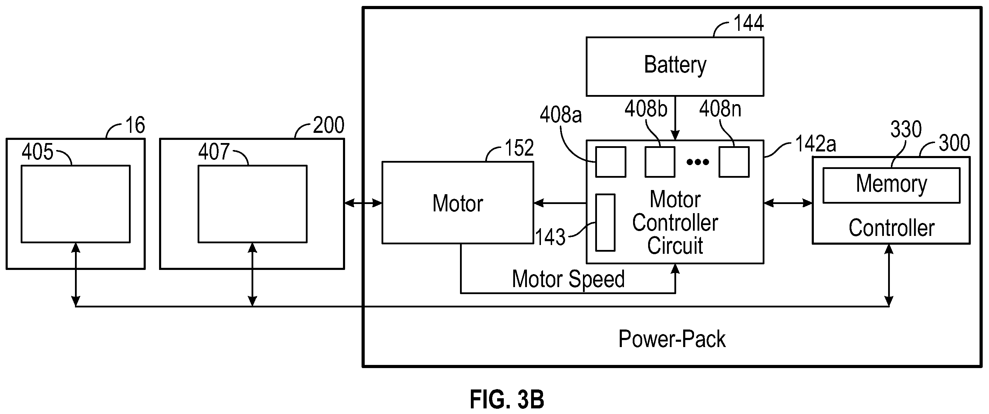

With reference to FIG. 3B, a schematic diagram of the handle assembly 20, the adapter assembly 200, and the reload assembly 16, is shown. For brevity, only one of the motors 152, 154, 156 is shown, namely, motor 152. The motor 152 is coupled to the battery 144. In embodiments, the motor 152 may be coupled to any suitable power source configured to provide electrical energy to the motor 152, such as an AC/DC transformer.

The battery 144 and the motor 152 are coupled to the motor controller circuit board 142a having a motor controller 143 which controls the operation of the motor 152 including the flow of electrical energy from the battery 144 to the motor 152. The main controller 300 (FIG. 3A) controls the handle assembly 20. The motor controller 143 includes a plurality of sensors 408a, 408b, ... 408n configured to measure operational states of the motor 152 and the battery 144. The sensors 408a-n may include voltage sensors, current sensors, temperature sensors, telemetry sensors, optical sensors, and combinations thereof. The sensors 408a-408n may measure voltage, current, and other electrical properties of the electrical energy supplied by the battery 144. The sensors 408a-408n may also measure angular velocity (e.g., rotational speed) as revolutions per minute (RPM), torque, temperature, current draw, and other operational properties of the motor 152. Angular velocity may be determined by measuring the rotation of the motor 152 or a drive shaft 106, 108, 110 (FIG. 2A) coupled thereto and rotatable by the motor 152. Positions of various axially movable drive shafts may also be determined by using various linear sensors disposed in or in proximity to the shafts or extrapolated from the RPM measurements. In aspects, torque may be calculated based on the regulated current draw of the motor 152 at a constant RPM. In further aspects, the motor controller 143 and/or the main controller 300 may measure time and process the above-described values as a function of time, including integration and/or differentiation, e.g., to determine the rate of change in the measured values. The main controller 300 is also configured to determine distance traveled of various components of the circular adapter assembly 200 and/or the reload assembly 16 by counting revolutions of the motors 152, 154, and 156.

The motor controller 143 is coupled to the main controller 300, which includes a plurality of inputs and outputs for interfacing with the motor controller 143. In particular, the main controller 300 receives measured sensor signals from the motor controller 143 regarding operational status of the motor 152 and the battery 144 and, in turn, outputs control signals to the motor controller 143 to control the operation of the motor 152 based on the sensor readings and specific algorithm instructions, which are discussed in more detail below. The main controller 300 is also configured to accept a plurality of user inputs from a user interface (e.g., switches, buttons, touch screen, etc. coupled to the main controller 300).

The main controller 300 is also coupled to the strain gauge 51 of the circular adapter assembly 200 using a wired or a wireless connection and is configured to receive strain measurements from the strain gauge 51 which are used during operation of the handle assembly 20.

The reload assembly 16 includes a storage device 405 (e.g., chip 464c). The adapter assembly 200 also includes a storage device 407. The storage devices 405 and 407 include non-volatile storage medium (e.g., EEPROM) that is configured to store any data pertaining to the reload assembly 16 and the circular adapter assembly 200, respectively, including but not limited to, usage count, identification information, model number, serial number, staple size, stroke length, maximum actuation force, minimum actuation force, factory calibration data, and the like. In aspects, the data may be encrypted and is only decryptable by devices (e.g., main controller 300) have appropriate keys. The data may also be used by the main controller 300 to authenticate the circular adapter assembly 200 and/or the reload assembly 16. The storage devices 405 and 407 may be configured in read only or read/write modes, allowing the main controller 300 to read as well as write data onto the storage device 405 and 407.

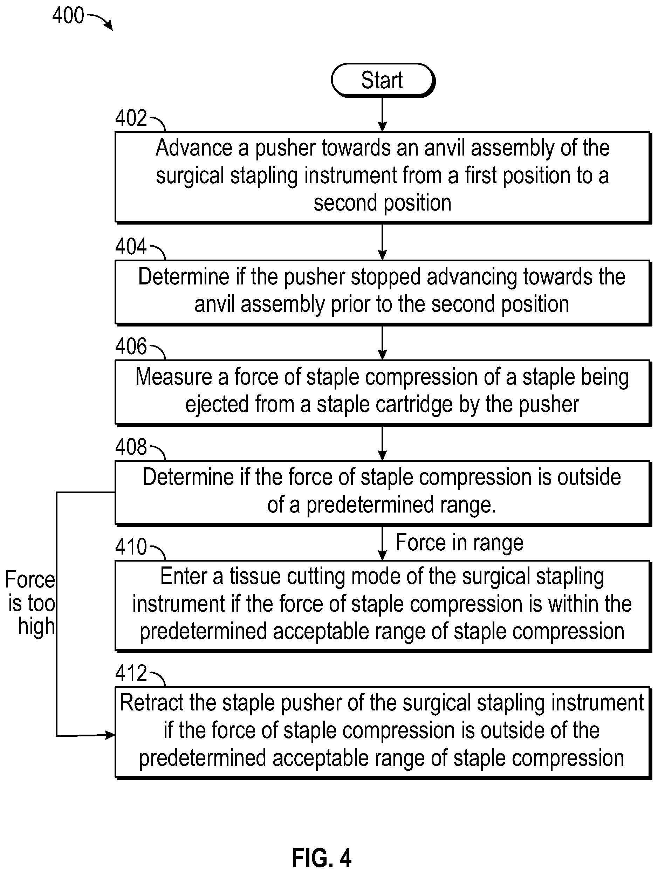

FIG. 4 illustrates a flow diagram of a computer-implemented method 400 for controlling a surgical stapling instrument 10 for determining whether a functionally closed staple formation has been achieved when current or force limits are measured. The method of forming an end to end anastomosis using the disclosed surgical stapling instrument 10 includes the clamping of tissue and the firing of staples "S" by the surgical stapling instrument 10 into the tissue. During the staple firing phase, the motor 154 drives the shaft 108 to move the drive assembly 119 through predetermined strokes (e.g., from a first position to a second position) to advance the pusher 61 within the shell housing 46 to eject staples "S" from the staple cartridge 48 (Step 402).

Once the pusher 61 is moved in relation to the anvil assembly 50, if the controller 300 determines that the pusher 61 has stopped advancing towards the anvil assembly 50 before it has reached a predetermined second position (Step 404), a force of staple compression on the staple "S" clamped between the staple cartridge 48 and the anvil assembly 50 is measured (Step 406). As discussed above, the stapling force of the staple "S" clamped between the anvil assembly 50 and the pusher 61 of the within the reload assembly 16 (FIG. 1) can be measured using the strain gauge 51 that communicates with the controller 300. Alternatively, other force or strain measuring devices may be used to measure the clamping pressure of the staple clamped between the anvil assembly 50 and the pusher 61. In various aspects, the current draw of the motor 154 may be used by the controller 300 to indicate the staple compression force.

The controller 300 determines if the staple compression force is outside of a predetermined acceptable range (Step 408). For example, the staple compression force may spike beyond the range of forces measured during staple formation.

If the compression force on the staple is within the predetermined acceptable range of compression, the surgical stapling instrument 10 enters the tissue cutting mode to allow a surgeon to cut the tissue and complete the procedure (Step 410). In aspects, the predetermined acceptable range of staple compression may vary depending on the type of tissue that is being treated and may be set automatically by the instrument 10 or by the user. In various aspects, the predetermined acceptable range of staple compression may be based on using different reloads, different staple heights, and/or different types of surgical staplers.

If the compression force is greater than the predetermined acceptable range of compression, the pusher 61 is retracted, and the surgical stapling instrument 10 exits firing mode (Step 412). In some aspects of the disclosure, the controller may provide a warning on a display 146 (FIG. 1) , *e.g.,* disposed the handle assembly of the surgical stapling instrument, to alert the surgeon that compression force on the tissue is not within the predetermined range of compression, such that the surgeon can reposition the surgical stapling instrument 10 on the tissue. In some aspects, the warning may be an audio alert, for example, a beep or a verbal warning to examine the surgical site.

Although this disclosure is directed to a powered surgical stapling instrument, it is envisioned the principles of this disclosure are applicable to manually powered stapling instruments. For example, the clamping pressure on tissue clamped between an anvil assembly and a staple cartridge of the stapling instrument can be measured as the stapling instrument is moved through a predetermined acceptable tissue gap range. In such a device, an indicator such as a light can be provided on the instrument. When the clamping pressure of the tissue enters the predetermined acceptable range of compression with the instrument within the predetermined acceptable gap range, the indicator can be activated to notify the surgeon that the instrument is ready to be fired.

It is envisioned that the aspects of this disclosure, although illustrated in association with a circular stapling instrument, are equally applicable to other types of stapling instruments, including linear stapling devices, vessel sealing devices, and other devices for joining tissue sections together.

Persons skilled in the art will appreciate that one or more operations of the method 500 may be performed in a different order, repeated, and/or omitted without departing from the scope of the disclosure. In various aspects, the illustrated method 400 can operate in the controller 300 (FIG. 3A), in a remote device, or in another server or system. Other variations are contemplated to be within the scope of the disclosure. The operations of method 400 are described with respect to a controller, *e.g*., controller 300 (FIG. 3A) of surgical stapling instrument 10 (FIG. 3A), but it will be understood that the illustrated operations are applicable to other systems and components thereof as well.

Persons skilled in the art will understand that the instruments and methods specifically described herein and illustrated in the accompanying drawings are non-limiting. It is envisioned that the elements and features may be combined with the elements and features of another without departing from the scope of the disclosure. As well, one skilled in the art will appreciate further features and advantages of the disclosure.

## Claims

1. A surgical stapling instrument comprising:
an anvil assembly including an anvil head and an anvil center rod extending proximally from the anvil head;
a reload assembly including a pusher and an annular staple cartridge including a plurality of staples, wherein the pusher is configured to eject staples from the annular staple cartridge;
a processor; and
a memory, including instructions stored thereon, **characterised in that**, which, when executed, cause the surgical stapling instrument to:
advance the pusher towards the anvil assembly from a first position to a second position;
determine if the pusher stopped advancing towards the anvil assembly prior to the second position;
measure a force of staple compression of a staple being ejected from the annular staple cartridge by the pusher, in response to the pusher having stopped advancing towards the anvil assembly; and
determine if the force of staple compression is outside of a predetermined range.

2. The surgical stapling instrument of claim 1, wherein the instructions, when executed by the processor, further cause the surgical stapling instrument to enter a tissue cutting mode of the surgical stapling instrument in response to the force of staple compression based on a predetermined acceptable range of staple compression.

3. The surgical stapling instrument of claim 1 or claim 2, wherein the force of staple compression is measured by a strain gauge; and/or wherein the force of staple compression is measured based on a current of a motor configured to advance the pusher.

4. The surgical stapling instrument of any of claims 1 to 3, wherein the instructions, when executed by the processor, further cause the surgical stapling instrument to prevent staple firing in response to the force of staple compression being greater than the predetermined range.

5. The surgical stapling instrument of any of claims 1 to 4 , wherein the instructions, when executed by the processor, further cause the surgical stapling instrument to display a warning on a display if the force of staple compression is greater than the predetermined range; preferably wherein the displayed warning includes at least one of a warning to inspect a surgical site or to unclamp tissue.

6. The surgical stapling instrument of any of claims 1 to 5, wherein the instructions, when executed by the processor, further cause the surgical stapling instrument to retract the pusher.

7. The surgical stapling instrument of any of claims 1 to 6, wherein the instructions, when executed by the processor, further cause the surgical stapling instrument to generate an audio warning in response to the force of staple compression being greater than the predetermined range.

8. A non-transitory computer-readable medium storing instructions which, when executed by a processor, cause the processor to perform a method for controlling a surgical stapling instrument, **characterised in that**, the method comprises:
advancing a pusher towards an anvil assembly of the surgical stapling instrument from a first position to a second position, the pusher configured to eject staples from a staple cartridge of the surgical stapling instrument;
determining if the pusher stopped advancing towards the anvil assembly prior to the second position;
measuring a force of staple compression of a staple being ejected from the staple cartridge by the pusher, in response to the pusher having stopped advancing towards the anvil assembly; and
determining if the force of staple compression is outside of a predetermined range.

## Patentansprüche

1. Chirurgisches Klammerinstrument umfassend:
eine Ambossbaugruppe, die einen Ambosskopf und eine Ambossmittelstange, die sich proximal von dem Ambosskopf erstreckt, aufweist;
eine Nachladebaugruppe aufweisend einen Schieber und ein ringförmiges Klammermagazin, das eine Vielzahl von Klammern enthält, wobei der Schieber dafür gestaltet ist, Klammern aus dem ringförmigen Klammermagazin auszustoßen;
einen Prozessor; und
einen Speicher, der darauf gespeicherte Anweisungen enthält, **dadurch gekennzeichnet, dass** sie, wenn ausgeführt, das chirurgische Klammerinstrument dazu veranlassen:
den Schieber von einer ersten Position in eine zweite Position in Richtung zu der Ambossanordnung vorzuschieben;
zu bestimmen, ob der Schieber vor der zweiten Position gestoppt hat, sich in Richtung zu der Ambossanordnung vorzuschieben;
eine Klammerkompressionskraft einer Klammer, die durch den Schieber aus dem ringförmigen Klammermagazin ausgestoßen wird, als Reaktion darauf zu messen, dass der Schieber gestoppt hat, sich in Richtung zu der Ambossanordnung vorzuschieben; und
zu bestimmen, ob die Klammerkompressionskraft außerhalb eines vorgegebenen Bereichs liegt.

2. Chirurgisches Klammerinstrument nach Anspruch 1, wobei die Anweisungen, wenn von dem Prozessor ausgeführt, ferner bewirken, dass das chirurgische Klammerinstrument als Reaktion auf die Klammerkompressionskraft auf der Grundlage eines vorgegebenen annehmbaren Bereichs von Klammerkompression in einen Gewebeschneidmodus des chirurgischen Klammerinstruments eintritt.

3. Chirurgisches Klammerinstrument nach Anspruch 1 oder Anspruch 2, wobei die Klammerkompressionskraft durch einen Dehnungsmessser gemessen wird; und/oder wobei die Klammerkompressionskraft auf der Grundlage eines Stroms eines Motors, der zum Vorschieben des Schiebers gestaltet ist, gemessen wird.

4. Chirurgisches Klammerinstrument nach einem der Ansprüche 1 bis 3, wobei die Anweisungen, wenn von dem Prozessor ausgeführt, ferner bewirken, dass das chirurgische Klammerinstrument Ausstoßen von Klammern als Reaktion darauf verhindert, dass die Klammerkompressionskraft größer als der vorgegebene Bereich ist.

5. Chirurgisches Klammerinstrument nach einem der Ansprüche 1 bis 4, wobei die Anweisungen, wenn von dem Prozessor ausgeführt, ferner bewirken, dass das chirurgische Klammerinstrument eine Warnung auf einer Anzeige anzeigt, wenn die Klammerkompressionskraft größer als der vorgegebene Bereich ist; wobei die angezeigte Warnung vorzugsweise wenigstens eine Warnung zum Inspizieren einer Operationsstelle oder zum Ausspannen von Gewebe enthält.

6. Chirurgisches Klammerinstrument nach einem der Ansprüche 1 bis 5, wobei die Anweisungen, wenn von dem Prozessor ausgeführt, ferner bewirken, dass das chirurgische Klammerinstrument den Schieber zurückzieht.

7. Chirurgisches Klammerinstrument nach einem der Ansprüche 1 bis 6, wobei die Anweisungen, wenn von dem Prozessor ausgeführt, ferner bewirken, dass das chirurgische Klammerinstrument eine akustische Warnung als Reaktion darauf erzeugt, dass die Klammerkompressionskraft größer als der vorgegebene Bereich ist.

8. Nichtflüchtiges computerlesbares Medium, das Anweisungen speichert, die bei Ausführung durch einen Prozessor bewirken, dass der Prozessor ein Verfahren zum Steuern eines chirurgischen Klammerinstruments ausführt, **dadurch gekennzeichnet, dass** das Verfahren umfasst:
Vorschieben eines Schiebers in Richtung zu einer Ambossanordnung des chirurgischen Klammerinstruments von einer ersten Position zu einer zweiten Position, wobei der Schieber dafür gestaltet ist, Klammern aus einem Klammermagazin des chirurgischen Klammerinstruments auszustoßen;
Bestimmen, ob der Schieber vor der zweiten Position gestoppt hat, sich in Richtung zu der Ambossanordnung vorzuschieben;
Messen einer Klammerkompressionskraft einer Klammer, die von dem Schieber aus dem Klammermagazin ausgestoßen wird, als Reaktion darauf, dass der Schieber gestoppt hat, sich in Richtung zu der Ambossanordnung vorzuschieben; und
Bestimmen, ob die Klammerkompressionskraft außerhalb eines vorgegebenen Bereichs liegt.

## Revendications

1. Instrument d'agrafage chirurgical comprenant :
un ensemble enclume comprenant une tête d'enclume et une tige centrale d'enclume s'étendant proximalement à partir de la tête d'enclume ;
un ensemble de recharge comprenant un poussoir et une cartouche d'agrafes annulaire comprenant une pluralité d'agrafes, le poussoir étant configuré pour éjecter des agrafes de la cartouche d'agrafes annulaire ;
un processeur ; et
une mémoire, comprenant des instructions stockées sur celle-ci, **caractérisé en ce que** les instructions, lorsqu'elles sont exécutées, amènent l'instrument d'agrafage chirurgical à :
avancer le poussoir vers l'ensemble enclume d'une première position à une seconde position ;
déterminer si le poussoir a cessé d'avancer vers l'ensemble enclume avant la seconde position ;
mesurer une force de compression d'agrafe d'une agrafe en cours d'éjection de la cartouche d'agrafes annulaire par le poussoir, en réponse au fait que le poussoir a cessé d'avancer vers l'ensemble enclume ; et
determiner si la force de compression d'agrafe est en dehors d'une plage prédéterminée.

2. Instrument d'agrafage chirurgical selon la revendication 1, les instructions, lorsqu'elles sont exécutées par le processeur, amenant en outre l'instrument d'agrafage chirurgical à entrer dans un mode de coupe de tissu de l'instrument d'agrafage chirurgical en réponse à la force de compression d'agrafe sur la base d'une plage de compression d'agrafe acceptable prédéterminée.

3. Instrument d'agrafage chirurgical selon la revendication 1 ou la revendication 2, la force de compression d'agrafe étant mesurée par une jauge de contrainte ; et/ou la force de compression d'agrafe étant mesurée sur la base d'un courant d'un moteur configuré pour faire avancer le poussoir.

4. Instrument d'agrafage chirurgical selon l'une quelconque des revendications 1 à 3, les instructions, lorsqu'elles sont exécutées par le processeur, amenant en outre l'instrument d'agrafage chirurgical à empêcher le tir d'agrafes en réponse au fait que la force de compression d'agrafe est supérieure à la plage prédéterminée.

5. Instrument d'agrafage chirurgical selon l'une quelconque des revendications 1 à 4, les instructions, lorsqu'elles sont exécutées par le processeur, amenant en outre l'instrument d'agrafage chirurgical à afficher un avertissement sur un affichage si la force de compression d'agrafe est supérieure à la plage prédéterminée ; de préférence, l'avertissement affiché comprenant au moins l'un parmi un avertissement d'inspecter un site chirurgical ou de desserrer un tissu.

6. Instrument d'agrafage chirurgical selon l'une quelconque des revendications 1 à 5, les instructions, lorsqu'elles sont exécutées par le processeur, amenant en outre l'instrument d'agrafage chirurgical à rétracter le poussoir.

7. Instrument d'agrafage chirurgical selon l'une quelconque des revendications 1 à 6, les instructions, lorsqu'elles sont exécutées par le processeur, amenant en outre l'instrument d'agrafage chirurgical à générer un avertissement audio en réponse au fait que la force de compression d'agrafe est supérieure à la plage prédéterminée.

8. Support non transitoire lisible par ordinateur stockant des instructions qui, lorsqu'elles sont exécutées par un processeur, amènent le processeur à mettre en œuvre un procédé de commande d'un instrument d'agrafage chirurgical **caractérisé en ce que** le procédé comprend :
l'avancement d'un poussoir vers un ensemble enclume de l'instrument d'agrafage chirurgical d'une première position à une seconde position, le poussoir étant configuré pour éjecter des agrafes d'une cartouche d'agrafes de l'instrument d'agrafage chirurgical ;
la détermination du fait que le poussoir a cessé d'avancer vers l'ensemble enclume avant la seconde position ;
la mesure d'une force de compression d'agrafe d'une agrafe en cours d'éjection de la cartouche d'agrafes par le poussoir, en réponse au fait que le poussoir a cessé d'avancer vers l'ensemble enclume ; et
la détermination si la force de compression d'agrafe est en dehors d'une plage prédéterminée.
